# EUROPEAN PATENT SPECIFICATION

(11) **EP 0 700 465 B1**
(45) Date of publication and mention of the grant of the patent: **07.04.1999**
(21) Application number: 94917406.4
(22) Date of filing: 17.05.1994
(51) Int. Cl.: D04H 13/00

(54) **PERSONAL CARE ARTICLE COMPRISING A LIGHTWEIGHT NONWOVEN WEB LAMINATE WITH IMPROVED COMFORT AND BARRIER PROPERTIES**
HYGIENEARTIKEL MIT LEICHTEM VERBUNDVLIESSTOFF MIT VERBESSERTER GESCHMEIDIGKEIT UND DICHTIGKEITSEIGENSCHAFTEN
ARTICLE D'HYGIENE COMPRENANT DE NON-TISSE LEGER MULTICOUCHE EN BANDES CONTINUES AYANT DES PROPRIETES AMELIOREES DU POINT DE VUE DU CONFORT ET DE L'EFFET BARRIERE

(30) Priority: 20.05.1993 US 65210; 02.03.1994 US 205684
(43) Date of publication of application: 13.03.1996
(73) Proprietor: KIMBERLY-CLARK WORLDWIDE, INC., Neenah, Wisconsin 54956 (US)
(72) Inventor: BUTT, Jon, Richard, Sr., Woodstock, GA 30188 (US); CREAGAN, Christopher, Cosgrove, Marietta, GA 30062 (US); DUNKERLY, Cedric, Arnett, II, Alpharetta, GA 30202 (US); YEO, Richard, Swee-chye, Medford, New Jersey 08055 (US)
(74) Representative: Diehl, Hermann, Dr. Dipl.-Phys.
(86) International application number: US9405462
(87) International publication number: WO9428224

(56) References cited:
- EP-A- 0 370 835
- EP-A- 0 569 860
- GB-A- 2 126 162
- US-A- 4 041 203
- US-A- 4 766 029

## Description

### Background of the Invention

Nonwoven fabric laminates are useful for a wide variety of applications. Such nonwoven fabric laminates are useful for wipers, towels, industrial garments, medical garments, medical drapes, and the like. In heavier basis weights the laminates are used in recreational applications such as tents and as car covers. Disposable fabric laminates have achieved especially widespread use in hospital operating rooms for drapes, gowns, towels, footcovers, sterilization wraps, and the like. Such surgical fabric laminates are generally spunbonded/meltblown/spunbonded (SMS) laminates consisting of nonwoven outer layers of spun-bonded polyolefins and an interior barrier layer of melt-blown polyolefins. Particularly, Kimberly-Clark Corporation, the assignee of the present invention, has for a number of years manufactured and sold SMS nonwoven surgical fabric laminates, sterilization wrap and recreational fabrics under the marks Spunguard® and Evolution® Such SMS fabric laminates have outside spunbonded layers which are durable and an internal melt-blown barrier layer which is porous but which, in combination with the spunbond layers, inhibits the strikethrough of fluids or the penetration of bacteria from the outside of the fabric laminate to the inside. In order for such a medical fabric to perform properly, it is necessary that the melt-blown barrier layer have a fiber size and a porosity that assures breathability of the fabric while at the same time inhibiting strikethrough of fluids.

Personal care absorbent articles such as disposable diapers, training pants, incontinent wear and feminine hygiene products utilize nonwoven fabrics for many purposes such as liners, transfer layers, absorbent media, backings, and the like. For many such applications the barrier properties of the nonwoven play an important role such as, for example, containment flaps described in coassigned U.S. Patent 4, 704, 116 to Enloe dated 3 November 1987, incoporated herein in its entirety by reference. It is also desirable for personal care products such as containment flaps that the nonwoven fabric be soft and conformable and that the porosity of the fabric provide a level of breathability for increased comfort. As cost is always a factor, the ability to provide these benefits at low cost is another consideration.

Although nonwoven laminates having some combination of the properties desired have been available, they have not been widely utilized for applications such as the aforementioned flaps because one or more of the important considerations has been lacking or not present to a desired degree. The present invention is directed to improved nonwoven laminates satisfying those and other desired requirements.

Other objects and advantages of the invention will become apparent upon reading the following detailed description and upon reference to the drawings.

### Summary

The present invention is directed to a personal care article comprising an improved lightweight nonwoven laminate including at least one fine fiber component layer and at least one continuous filament layer. The fine fiber layer includes fibers having an average diameter in the range of up to about 10 µm (microns) and a basis weight in the range of from about 3 g/m² (gsm) to about 26 g/m² (gsm). The continuous filament web has filaments with an average diameter in the range of from about 12 µm (microns) to about 22 µm (microns) and a basis weight in the range of from about 10g/m² (gsm) to about 30 g/m² (gsm). The layers are bonded intermittently for a total basis weight non to exceed about 55 g/m² (gsm) and with the ratio of fine fibers to continuous filaments at least 20%. The resulting laminate has an improved combination of properties including softness and conformability as measured by a cup crush peak load test value no more than 150 g, cup crush test energy value of no more than 2250g/mm, barrier as measured by hydrostatic head of at least 15 cm, and breathability as measured in terms of Frazier porosity of at least 0.0236 m³/s (50 scfm). Preferred embodiments include spunbond continuous filament webs and meltblown fine fiber webs as the respective layers.

### Brief Description of the Drawings

Figure 1 is a schematic diagram of a forming machine which is used in making the nonwoven fabric laminate including the melt-blown barrier layer of the present invention;
Figure 2 is a cross-section view of the nonwoven fabric laminate of the present invention showing the layer configuration including the internal fine fiber barrier layer made in accordance with the present invention;
Figure 3 is a cross-section view of an alternative embodiment of the nonwoven fabric laminate of the present invention in a two layer configuration; and
Figure 4 is a cross-section view of a third embodiment of the nonwoven fabric laminate of the present invention with external fine fiber layers.

### Detailed Description of the Invention

While the invention will be described in connection with a preferred embodiment, it will be understood that we do not intend to limit the invention to that embodiment.

The present invention is directed to a personal care article comprising an improved lightweight nonwoven laminate including at least one fine fiber component layer and at least one continuous filament layer. The fine fiber layer includes fibers having an average diameter in the range of up to about 10 µm (microns) and a basis weight in the range of from about 3 g/m² (gsm) to about 26 g/m² (gsm). The continuous filament web has filaments with an average diameter in the range of from about 12 µm (microns) to about 22 µm (microns) and a basis weight in the range of from about 10 g/m² (gsm) to about 30 g/m² (gsm). The layers are bonded intermittently for a total basis weight not to exceed about 55 g/m² (gsm) and with the amount of fine fibers based on the laminate weight of at least 20%. The resulting laminate has an improved combination of properties including softness and conformability as measured by a cup crush test value no more than 150 g, barrier as measured by hydrostatic head of at least 15 cm, and breathability as measured in terms of Frazier porosity of at least 0.0236 m³/s (50 scfm). Preferred embodiments include spunbond continuous filament webs and meltblown fine fiber webs as the respective layers.

The foregoing objectives are preferably obtained by forming a melt-blown web in accordance with U.S. Patent 5,213,881 dated 25 May 1993, incorporated herein in its entirety by reference, from a propylene polymer resin having a broad molecular weight distribution and having a high melt flow rate which resin is modified by the addition of a small amount of peroxide prior to processing to achieve an even higher melt flow rate (lower viscosity). In general, the present invention involves starting with a propylene polymer in the form of reactor granules which polymer has a molecular weight distribution of 3.6 to 4.8 Mw/Mn, preferably 3.6 to 4.0 Mw/Mn and a melt flow rate of about 400 g/m²/10 min (gms/10 min) to 3000 g/m²/10 min (gms/10 min) at 230°C. Such a molecular weight reactor granule polymer is then modified to reduce and narrow the polymer's molecular weight distribution to a range from 2.2 to 3.5 Mw/Mn by the addition of up to 3000 parts per million (ppm) of peroxide. During the meltblowing process, the modified reactor granule polymer has an increased melt flow rate from 400 g/m²/10 min (gms/10 min) to 3000, for example, to a range between 800 up to 5000 g/m²/10 min (gms/10 min) at 230°C.

Particularly preferred embodiments include a polypropylene resin in the form of a reactor granule having a starting molecular weight distribution of 3.6 to 4.8 Mw/Mn and a melt flow rate of from 600 to 3000 g/m²/10 min (gms/10 min) at 230°C which is combined with a small amount of peroxide, less than 500 ppm, to produce a modified polypropylene having a very high melt flow rate of up to 5000 g/m²/10 min (gms/10 min) at 230°C and a narrower molecular weight distribution of 2.8 to 3.5 Mw/Mn.

Alternatively, an improved fine fiber web for use as a barrier layer can be formed by utilizing a resin, particularly polypropylene, having a narrow molecular weight distribution and having a lower melt flow rate which resin is modified by the addition of a larger amount of peroxide prior to melt-blowing to achieve a high melt flow rate. The starting reactor granule polypropylene resin in this case has a molecular weight distribution between 4.0 and 4.8 Mw/Mn and a melt flow rate ranging from 400 to 1000 g/m²/10 min (gms/10 min) at 230°C. The polypropylene resin is modified by adding peroxide in amounts ranging from 500 to 3000 ppm (the higher amounts of peroxide being used in connection with the lower initial melt flow rate). The modified polypropylene resin has a melt flow rate, up to about 3000 g/m²/10 min (gms/10 min) at 230°C and a narrow molecular weight distribution of 2.2 to 2.8 Mw/Mn, for example.

Most preferably, the starting polypropylene resin for the fine fiber web of the lightweight nonwoven laminate of the present invention is a polypropylene reactor granule which resin has a molecular weight distribution between 3.6 and 4.8 Mw/Mn, has a melt flow rate of up to 3000 g/m²/10 min (gms/10 min) at 230°C, and is treated with about 500 ppm of peroxide to produce a modified resin having a melt flow rate greater than 2000 g/m²/10 min (gms/10 min) at 230°C and a molecular weight distribution of from 2.8 to 3.5 Mw/Mn. The broader molecular weight distribution at the high melt flow rate helps minimize production of lint and polymer droplets (shot).

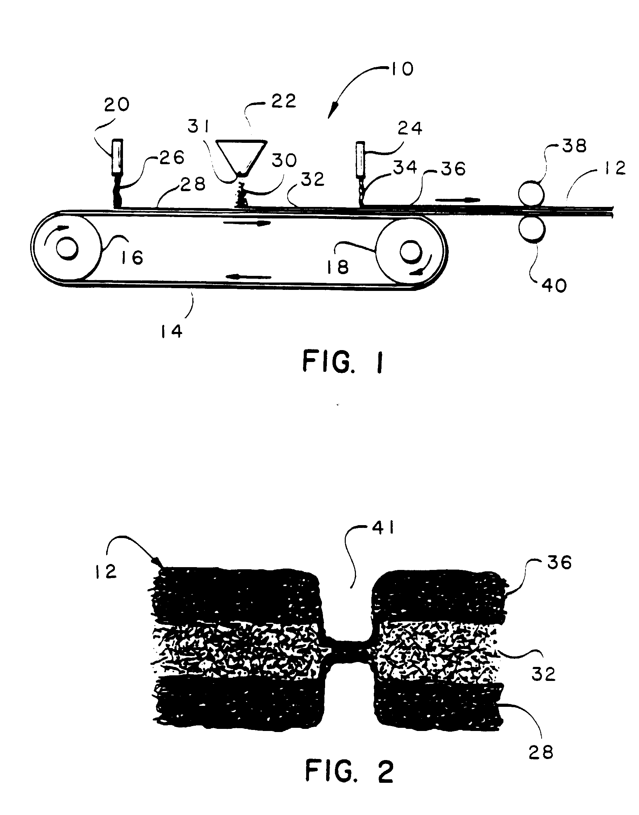

Turning to Figure 1, there is shown schematically a forming machine 10 which may be used to produce an improved nonwoven fabric laminate 12 having a fine fiber meltblown barrier layer 32 in accordance with the present invention. Particularly, the forming machine 10 consists of an endless foraminous forming belt 14 wrapped around rollers 16 and 18 so that the belt 14 is driven in the direction shown by the arrows. The forming machine 10 has three stations, spun-bond station 20, melt-blown station 22, and spun-bond station 24. It should be understood that more than three forming stations may be utilized to build up layers of higher basis weight. Alternatively, each of the laminate layers may be formed separately, rolled, and later converted to the fabric laminate off-line. In addition the fabric laminate 12 could be formed of more than or less than three layers depending on the requirements for the particular end use for the fabric laminate 12. For example, for some applications it may be preferred to have at least two inner meltblown layers for improved performance and for extremely lightweight applications a two-layer laminate may be made.

The spunbond stations 20 and 24 are conventional extruders with spinnerettes which form continuous filaments of a polymer and deposit those filaments onto the forming belt 14 in a random interlaced fashion. The spun-bond stations 20 and 24 may include one or more spinnerette heads depending on the speed of the process and the particular polymer being used. Forming spunbonded material is conventional in the art, and the design of such a spunbonded forming station is thought to be well within the ability of those of ordinary skill in the art. The nonwoven spunbonded webs 28 and 36 are prepared in conventional fashion such as illustrated by the following patents: Dorschner et al. United States Patent No 3,692,618; Kinney United States Patent Nos. 3,338,992 and 3,341,394; Levy United States Patent No. 3,502,538; Hartmann United States Patent Nos. 3,502,763 and 3,909,009; Dobo et al. United States Patent No. 3,542,615; Harmon Canadian Patent No. 803,714; and Appel et al. United States Patent No. 4,340,563. Other methods for forming a nonwoven web having continuous filaments of a polymer are contemplated for use with the present invention.

Spunbonded materials prepared with continuous filaments generally have at least three common features. First, the polymer is continuously extruded through a spinnerette to form discrete filaments. Thereafter, the filaments are drawn either mechanically or pneumatically without breaking In order to molecularly orient the polymer filaments and achieve tenacity. Lastly, the continuous filaments are deposited in a substantially random manner onto a carrier belt to form a web. Particularly, the spunbond station 20 produces spun-bond filaments 26 from a fiber forming polymer. The filaments are randomly laid on the belt 14 to form a spunbonded external layer 28. The fiber forming polymer is described in greater detail below.

The meltblown station 22 consists of a die 31 which is used to form microfibers 30. The throughput of the die 31 is specified in kg of polymer melt per cm of die width per seconds (pounds of polymer melt per inch of die width per hour (PIH)). As the thermoplastic polymer exits the die 31, high pressure fluid, usually air, attenuates and spreads the polymer stream to form microfibers 30. The microfibers 30 are randomly deposited on top of the spunbond layer 28 and form a meltblown layer 32. The construction and operation of the meltblown station 22 for forming microfibers 30 and meltblown layer 32 is considered conventional, and the design and operation are well within the ability of those of ordinary skill in the art. Such skill is demonstrated by NRL Report 4364, "Manufacture of Super-Fine Organic Fibers", by V.A. Wendt, E.L. Boon, and C.D. Fluharty; NRL Report 5265, "An Improved Device for the Formation of Super-Fine Thermoplastic Fibers", by K.D. Lawrence, R.T. Lukas, and J.A. Young; and United States Patent No. 3,849,241, issued November 19, 1974, to Buntin et al. Other methods for forming a nonwoven web of microfibers are contemplated for use with the present invention.

The meltblown station 22 produces fine fibers 30 from a fiber forming polymer which will be described in greater detail below. The fibers 30 are randomly deposited on top of spunbond layer 28 to form a meltblown internal layer 32. For a barrier flap fabric laminate, for example, the meltblown barrier layer 32 has a basis weight of preferably about 3 gsm to about 26 g/m² (gsm), more preferably from about 6 g/m² (gsm) to about 12 g/m² (gsm).

After the internal layer 32 has been deposited by the meltblown station 22 onto layer 28, spun-bond station 24 produces spunbond filaments 34 which are deposited in random orientation on top of the melt-blown layer 32 to produce external spunbond layer 36. For a barrier flap fabric laminate, for example, the layers 28 and 36 each have a basis weight of preferably from about 10 g/m² (gsm) to about 30 g/m² (gsm), more preferably about 15 g/m² (gsm) to about 25 g/m² (gsm).

The resulting SMS fabric laminate web 12 (Fig. 2) is then fed through bonding rolls 38 and 40. The surface of the bonding rolls 38 and 40 are provided with a raised pattern such as spots or grids. The bonding rolls are heated to the softening temperature of the polymer used to form the layers of the web 12. As the web 12 passes between the heated bonding rolls 38 and 40, the material is compressed and heated by the bonding rolls in accordance with the pattern on the rolls to create a pattern of discrete areas, such as 41 shown in Fig. 2, which areas are bonded from layer to layer and are bonded with respect to the particular filaments and/or fibers within each layer. Such discrete area or spot bonding is well-known in the art and can be carried out as described by means of heated rolls or by means of ultrasonic heating of the web 12 to produced discrete area thermally bonded filaments, fibers, and layers. In accordance with conventional practice described in Brock et al., United States Patent No. 4,041,203, it is preferable for the fibers of the meltblown layer in the fabric laminate to fuse within the bond areas while the filaments of the spun-bonded layers retain their integrity in order to achieve good strength characteristics. For heavier basis weight laminates, for example, sonic bonding as described in United States Patent 4,374,888, incorporated herein by reference, is preferred.

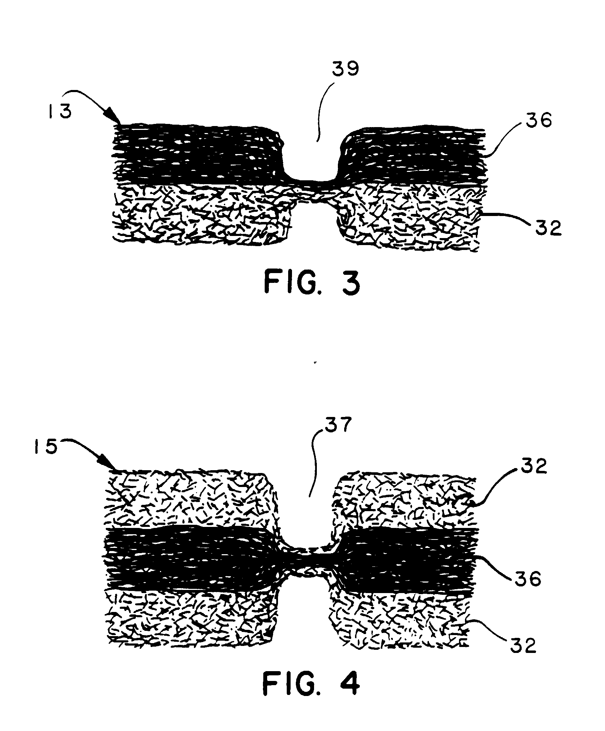

Turning to Figs. 3 and 4, alternative embodiments are illustrated. Fig. 3 is a cross-section similar to Fig. 2 showing a two layer laminate 13 comprised of fine fiber layer 32 and continuous filament layer 36 combined by thermal bond 39. Fig. 4 is a similar view of an alternative three-layer embodiment 15 comprising outer fine fiber layers 32 with inner continuous filament layer 36 combined by thermal bond 37.

In accordance with the invention, the total basis weight of the laminate is in the range of up to about 55 g/m² (gsm), more preferably up to about 34 g/m² (gsm), most preferably up to about 29 g/m² (gsm) and the amount of fine fibers compared to continuous filaments is at least about 20%, more preferably at least about 25% based on total weight of fine fibers and continuous filaments.

In accordance with the present invention, a preferred embodiment of a meltblown web formed in accordance with U.S. Patent 5,213,881 to Timmons, Kobylivker and Woon dated 25 May 1993, incorporated herein by reference, is utilized as the fine fiber component or components.

The resulting meltblown web 32 with its fine fibers and resulting small pore size distribution has superior barrier properties when incorporated into a fabric laminate. Particularly, the unlaminated meltblown web 32 has an average fiber size of from 1 to 3 µm (microns) and pore sizes distributed predominantly in the range from 7 to 12 µm (microns), with a lesser amount of pores from 12 to 25 µm (microns), with virtually no pores greater than 25 µm (microns) , and with the peak of the pore size distribution less than 10 µm (microns).

The present invention can be carried out with polyolefins including predominantly propylene polymer but which may include, polyethylene, or other alphaolefins polymerized with Ziegler-Natta catalyst technology, and copolymers, terpolymers, or blends thereof. Polypropylene is preferred for the continuous filament web.

### EXAMPLE

A lightweight nonwoven laminate was produced generally in accordance with the teachings of U.S. Patent 4,041,203 to Brock and Meitner dated 9 August 1977, incorporated herein in its entirety by reference. An in-line process was utilized as shown in Fig. 1 where the initial layer of spunbond is laid on the forming wire followed by the meltblown layer and finally the final layer of spunbond. The target total basis weight of the fabric was between 25 g/m² (gsm) and 34 g/m² (gsm) with the meltblown making up from between 6 g/m² (gsm) and 12 g/m² (gsm) of the total. For this Example, equal amounts of spunbond were on each side of the meltblown web although non essential to the invention.

The three-layer laminate material was then bonded using a thermal-mechanical bonder as in the above-mentioned U.S. Patent 4,041,203. As is preferred, a pattern bond roll with a percent bond area from 5 to 20%, target of 13%, and with a pin density from 7.75 to 54 pin/cm² (50 to 350 pin/sq. in.), target of 46.5 pin/cm² (300/sq. in.) was utilized. The temperature system was between 93°C to 149°C (200°F to 300°F) with a target of 121.1°C (250°F). Bonding pressure was set so that a uniform nip was maintained across the face of the unit.

In accordance with the foregoing an in-line SMS fabric was produced with a total weight of 29 g/m² (gsm) of which 25% was made up of meltblown. The spunbond polymer was Exxon PD3445 polypropylene and the meltblown was Exxon 3495G polypropylene, both of which are available from Exxon Chemical Company. The fabric was then bonded using a "wire weave" pattern roll that had a bond area of 13% with a pin density of 46.5 pin/cm² (300 pin/sq. in.) and was operated at a temperature of 121.1°C (250°F).

Table 1 illustrates the combination of properties obtained with the nonwoven laminate material of the Example. Basis weight was determined in accordance with ASTM Standard Test D3775-9. Hydrostatic head was determined in accordance with Method 5514 Federal Test Methods STD No. 191A, also AATCC STD 127-1980. Frazier air porosity was determined in accordance with ASTM D737, also Federal Test Methods 5450 Standard No. 191A. Cup crush results were determined by measuring the peak load required for a 4.5 cm diameter hemispherically shaped foot to crush a 9"x9" piece of fabric shaped into an approximately 6.5 cm diameter by 6.5 cm tall inverted cup while the cup shaped fabric was surrounded by an approximately 6.5 cm diameter cylinder to maintain a uniform deformation of the cup shaped fabric. The foot and the cup were aligned to avoid contact between the cup walls and the foot which could affect the peak load. The peak load was measured while the foot was descending at a rate of about 0.635 cm/s (0.25 inches per second (15 inches per minute)) utilizing a Model FTD-G-500 load cell (500 gram range) available from the Schaevitz Company, Tennsauken, New Jersey which provides the energy value.

**TABLE 1**

| Fine Fiber Layer | |
|---|---|
| Composition | polypropylene |
| Average fiber diameter (µm (microns)) | 3 |
| Basis weight (g/m² (gsm)) | 7. 2 |

| Continuous Filament Layers | |
|---|---|
| Composition | polypropylene |
| Average filament diameter (µm microns)) | 21 |
| Basis weight (g/m² (gsm-each)) | 10.8 |

| Laminate | |
|---|---|
| Basis weight (g/m² (gsm-each)) | 28.82 |
| Frazier Porosity | 0.035 m³/s (75 SCFM) |
| Hydrostatic head (cm water) | 45 |
| Cup Crush Peak Load (g) | 70 |
| Cup Crush Energy (g/mm) | 1500 |

When incorporated into a personal care article as a barrier flap component, the laminate of the Example demonstrated highly desired functionality and perceived comfort.

Thus, in accordance with the invention there has been described an improved lightweight nonwoven laminate. Variations and alternative embodiments will be apparent to those skilled in the art and are intended to be embraced within the appended claims.

## Claims

1. A personal care article comprising a nonwoven fabric laminate (12;13;15), the nonwoven fabric laminate (12;13;15) comprising:
a) a nonwoven component layer (32) comprising fine fibers having an average diameter in the range of up to about 10 µm (microns) and a basis weight in the range of from about 3 g/m² (gsm) to about 26 g/m² (gsm), and
b) a nonwoven component layer (36) comprising continuous filaments having an average diameter in the range of from about 12 µm (microns) to about 22 µm (microns) and a basis weight in the range of from about 10 g/m² (gsm) to about 30 g/m² (gsm),
wherein said layers (32;36) are intermittently bonded in a face-to-face relationship for a total basis weight not to exceed about 55 g/m² (gsm) and the percent of the weight of fine fibers layer to the laminate weight is at least 20%, and wherein said laminate (12;13;15) has a cup crush peak load value of no more than 150 grams, a cup crush energy value of no more than 2250 g/mm, a hydrostatic head of at least 15 cm, and a porosity of at least 0.0236 m³/s (50 scfm).

2. The personal care article of claim 1 wherein the total basis weight is in the range of up to about 34 g/m² (gsm).

3. The personal care article of claim 2 wherein the total basis weight is in the range of up to about 29 g/m² (gsm).

4. The personal care article of any of the preceding claims wherein the continuous filament component layer (36) comprises a propylene polymer.

5. The personal care article of any of the preceding claims wherein the fine fiber component layer (32) comprises a propylene polymer having a molecular weight distribution between 3.6 and 4.8 Mw/Mn and a melt flow rate up to about 3000 g/10 min. at 230°C.

6. The personal care article of any of the preceding claims comprising two continuous filament layers (36) on opposite sides of a fine fiber layer (32).

7. The personal care article of claim 6 wherein the ratio of the fine fibers to continuous filaments is at least 25%.

8. The personal care article of any of the preceding claims wherein the personal care article is a disposable diaper.

## Patentansprüche

1. Körperpflegeartikel, umfassend ein Vliesstofflaminat (12; 13; 15), wobei das Vliesstofflaminat (12; 13; 15) umfaßt:
a) eine Vlieskomponentenschicht (32), umfassend Feinfasern mit einem durchschnittlichen Durchmesser im Bereich von etwa 10 µm (Mikron) und einem Flächengewicht im Bereich von etwa 3 g/m² (gsm) bis etwa 26 g/m² (gsm), und
b) eine Vlieskomponentenschicht (36), umfassend Endlosfasern mit einem durchschnittlichen Durchmesser im Bereich von etwa 12 µm (Mikron) bis etwa 22 µm (Mikron) und einem Flächengewicht im Bereich von etwa 10 g/m² (gsm) bis etwa 30 g/m² (gsm),
wobei die Schichten (32; 36) satzweise in einer Vorderfläche-an-Vorderfläche-Beziehung bis zu einem Gesamtflächengewicht von nicht mehr als etwa 55 g/m² (gsm) miteinander verklebt sind und das Prozentverhältnis des Gewichts der Feinfaserschicht zum Laminatgewicht mindestens 20% beträgt, und wobei das Laminat (12; 13; 15) einen Becherbrechspitzenlastwert von nicht mehr als 150 Gramm, einen Becherbrechenergiewert von nicht mehr als 2250 g/mm, einen hydrostatischen Druck von mindestens 15 cm und eine Porosität von mindestens 0,0236 m²/s (50 scfm) aufweist.

2. Körperpflegeartikel nach Anspruch 1, wobei das Gesamtflächengewicht im Bereich von bis zu etwa 34 g/m² (gsm) liegt.

3. Körperpflegeartikel nach Anspruch 2, wobei das Gesamtflächengewicht im Bereich von bis zu etwa 29 g/m² (gsm) liegt.

4. Körperpflegeartikel nach einem der vorhergehenden Ansprüche, wobei die Endlosfaserkomponentenschicht (36) ein Propylenpolymer umfaßt.

5. Körperpflegeartikel nach einem der vorhergehenden Ansprüche, wobei die Feinfaserkomponentenschicht (32) ein Propylenpolymer mit einer Molekulargewichtverteilung zwischen 3,6 und 4,8 Mw/Mn und eine Schmelzflußrate von bis zu etwa 3000 g/10 Min. bei 230°C umfaßt.

6. Körperpflegeartikel nach einem der vorhergehenden Ansprüche, umfassend zwei Endlosfaserschichten (36) an gegenüberliegenden Seiten einer Feinfaserschicht (32).

7. Körperpflegeartikel nach Anspruch 6, wobei das Verhältnis zwischen den Feinfasern und den Endlosfasern mindestens 25% beträgt.

8. Körperpflegeartikel nach einem der vorhergehenden Ansprüche, wobei es sich bei dem Körperpflegeartikel um eine Wegwerfwindel handelt.

## Revendications

1. Article d'hygiène intime comprenant un stratifié d'étoffes non tissées (12;13;15), ledit stratifié d'étoffes non tissées (12;13;15) comprenant :
a) une couche constitutive non tissée (32) comprenant des fibres fines ayant un diamètre moyen pouvant aller jusqu'à environ 10 microns et un poids de base compris dans la gamme allant d'environ 3 g/m² à environ 26 g/m², et
b) une couche constitutive non tissée (36) comprenant des filaments continus ayant un diamètre moyen compris dans la gamme allant d'environ 12 microns à environ 22 microns et un poids de base compris dans la gamme allant d'environ 10 g/m² à environ 30 g/m²,
dans lequel lesdites couches (32;36) sont liées par intermittence selon une relation face-à-face pour avoir un poids de base total n'excédant pas environ 55 g/m² et le pourcentage du poids de la couche formée de fibres fines au poids du stratifié est d'au moins 20 %, et
dans lequel ledit stratifié (12;13;15) a une valeur de charge maximale d'écrasement de coupelle n'excédant pas 150 g, une valeur d'énergie d'écrasement de coupelle n'excédant pas 2 250 g/mm, une pression hydrostatique d'au moins 15 cm et une porosité d'au moins 0,0236 m³/s (50 pieds³ standard/minute).

2. Article d'hygiène intime selon la revendication 1, dans lequel le poids de base total peut aller jusqu'à environ 34 g/m².

3. Article d'hygiène intime selon la revendication 2, dans lequel le poids de base total peut aller jusqu'à environ 29 g/m².

4. Article d'hygiène intime selon l'une quelconque des revendications précédentes, dans lequel la couche constitutive de filaments continus (36) est constituée d'un polymère de propylène.

5. Article d'hygiène intime selon l'une quelconque des revendications précédentes, dans lequel la couche constitutive de fibres fines (32) est constituée d'un polymère de propylène ayant une distribution de masse molaire comprise entre 3,6 et 4,8 Mw/Mn et un débit d'écoulement à l'état fondu allant jusqu'à environ 3 000 g/10 minutes à 230°C.

6. Article d'hygiène intime selon l'une quelconque des revendications précédentes, comprenant deux couches de filaments continus (36) sur les faces opposées d'une couche de fibres fines (32).

7. Article d'hygiène intime selon la revendication 6, dans lequel le rapport des fibres fines aux filaments continus est d'au moins 25 %.

8. Article d'hygiène intime selon l'une quelconque des revendications précédentes qui est un change jetable.
